# EUROPÄISCHE PATENTSCHRIFT

(11) **EP 2 802 553 B1**
(45) Veröffentlichungstag und Bekanntmachung des Hinweises auf die Patenterteilung: **06.04.2016**
(21) Anmeldenummer: 13700480.0
(22) Anmeldetag: 04.01.2013
(51) Int. Cl.: C07C 209/16, C07C 209/86, C07C 211/08, C07C 211/35

(54) **VERFAHREN ZUR HERSTELLUNG VON SEKUNDÄREN AMINEN IN DER FLÜSSIGPHASE**
METHOD FOR PRODUCING SECONDARY AMINES IN THE LIQUID PHASE
PROCÉDÉ DE PRODUCTION D'AMINES SECONDAIRES EN PHASE LIQUIDE

(30) Priorität: 11.01.2012 EP 12150717
(43) Veröffentlichungstag der Anmeldung: 19.11.2014
(73) Patentinhaber: BASF SE, 67056 Ludwigshafen am Rhein (DE)
(72) Erfinder: WIGBERS, Christof Wilhelm, 68167 Mannheim (DE); MELDER, Johann-Peter, 67459 Böhl-Iggelheim (DE); STEIN, Bernd, 64665 Alsbach-Hähnlein (DE)
(86) Internationale Anmeldenummer: PCT/EP2013/050077
(87) Internationale Veröffentlichungsnummer: WO 2013/104560

(56) Entgegenhaltungen:
- EP-A1- 0 526 318
- EP-A2- 0 588 156
- WO-A1-2011/124577
- DE-A1- 3 641 666
- JP-A- 2011 026 214
- US-A- 4 206 150

## Beschreibung

Die vorliegende Anmeldung betrifft ein Verfahren zur Herstellung von sekundären Aminen durch Aminierung von überschüssigen primären oder sekundären Alkoholen mit primären Aminen in der Flüssigphase in Gegenwart von Kupfer enthaltenden Katalysatoren.

Sekundäre Amine stellen wichtige, technisch genutzte Stoffe dar. Sie dienen beispielsweise als Polymerisations- und Härtungskatalysatoren für die Herstellung von Kunststoffformkörpern auf der Basis von Epoxiden und Polyurethanen, als Korrosionsinhibitoren und als Ausgangsstoffe für Flockungsmittel und Detergenzien. Weiterhin werden sekundäre Amine als Zwischenprodukte im Pflanzenschutz verwendet.

Die Herstellung von sekundären Aminen durch Aminierung von Alkoholen oder Aldehyden mit primären Aminen in der Flüssig- und Gasphase und von Aldehyden mit primären Aminen in der Flüssigphase in Gegenwart von Wasserstoff und Hydrierkatalysatoren ist bekannt.

In JP 2011026214 wird die Umsetzung von primären Alkoholen mit C₁ bis C₃-Alkylgruppen in der Gasphase in Gegenwart von Kupfer enthaltenden Katalysatoren mit tert.-Butylamin zu N-Alkyl-tert.-butylamin beschrieben. In diesem Verfahren werden jedoch nur mäßige Selektivitäten und Ausbeuten erreicht. So wird bei der Herstellung von N-Ethyl-tert.-butylamin eine Ausbeute von nur 77 % und eine Selektivität von 80,6 % bei 250 °C in Gegenwart von CuO/ZnO-Katalysatoren erzielt. Des Weiteren wird in diesem Verfahren in der Gasphase gearbeitet, was einen höheren Energiebedarf verlangt und eine großtechnische Gewinnung von sekundären Aminen weniger günstig macht.

In US 4206150 werden als Vergleichsbeispiele 6 und 7 Verfahren zur Herstellung von sekundären Aminen beschrieben, bei denen 1-Dodecanol mit Monomethylamin oder Dimethylamin bei 200 °C und 375 psi. an einem Kupfer auf Al₂O₃ geträgerten Katalysator in der Flüssigphase umgesetzt werden, wobei der Kupfergehalt 10 bzw. 3,9 Gew.-% beträgt und das Amin im dreifachen Überschuss zum Alkohol eingesetzt wird. Nachteilig bei diesen Verfahren ist, zum einen der hohe Gehalt an Nebenprodukten (14,1 % C₂₄- + C₂₅-Amine) sowie die geringe Katalysatorstabilität, da dieser nach der Aminierung ausblutet. Die Lehre aus US 4206150 ist daher, dass für die Herstellung von sekundären Aminen ausgehend von primären oder sekundären Alkoholen mit Aminen in der Flüssigphase der Katalysator neben Kupfer auch Molybdän und/oder Wolfram in Form ihrer Metalle und/oder Oxide enthalten muss und das Amin im Überschuss zum Alkohol eingesetzt werden muss, damit der Katalysator über längere Zeit nicht auszublutet und gute Ausbeuten erzielt werden. Weiterhin ist auch die destillative Aufreinigung eines nach US 4,206,150 hergestellten sekundären Amins nicht einfach möglich, da es bei der Destillation zur Bildung von Homoazeotropen aus Amin und Wasser kommt, die sich destillativ nicht trennen lassen.

EP-B 257443 beschreibt ein Verfahren zur Herstellung von Trialkylaminen durch Umsetzung von primären Alkoholen mit Ammoniak oder einem primären Alkylamin in der Flüssigphase in Gegenwart von Kupferkatalysatoren mit Al₂O₃ als Träger bei Temperaturen im Bereich von 230°C bis 235°C bei dem nur sehr geringe Mengen and sekundären Aminen (ca. 3%) entstehen. EP 257443 offenbart weiterhin, dass bei der Verwendung von primären Aminen mit primären Alkoholen im Überschuss, einem Kupferkatalysator als Hydrierkatalysator bei Verwendung von Temperaturen unterhalb von 240°C bevorzugt tertiäre Alkylamine hergestellt werden können. Dass im Temperaturbereich von kleiner 210°C bevorzugt sekundäre Amine mit hoher Ausbeute und Selektivität hergestellt werden können, wird in EP-B 257443 nicht offenbart.

EP 588156 beschreibt die Umsetzung von Alkanolen mit Alkylaminen und Wasserstoff in Gegenwart von Kupferchromit/Erdalkalichromit-Katalysatoren bei 180 bis 210°C und 40 bis 120 bar in der Flüssigphase. Das Amin/Alkanol-Molverhältnis beträgt 1,5 zu eins bis 50 zu 1, so dass dieses Verfahren immer mit einem Überschuss an Amin arbeitet. Nachteilig bei diesem Verfahren ist die Verwendung von chromhaltigen und damit toxischen Katalysatoren.

Aus DE 364 1666 geht hervor, dass sich sekundäre Amine durch Umsetzung von Alkoholen mit einem Überschuss an primären Aminen bei 150 bis 250 °C und nur 1 bis 6 bar herstellen lassen. Hierfür sind Kupfer/Nickel-Katalysatoren notwendig, die zusätzlich ein Element aus der Platin-Gruppe, insbesondere Pt, Pd, Ru oder Rh enthalten. Nachteilig ist, zum einen der Einsatz von teuren Hydriermetallen wie Platin und zum anderen der Einsatz an überschüssigem Amin, da dies die anschließende destillative Trennung erschwert. Außerdem muss das Reaktionswasser intermittierend oder kontinuierlich aus dem Reaktionsgemisch entfernt werden.

EP 0526318 beschreibt die Herstellung von sekundären Aminen zwar in Gegenwart eines Überschusses an Alkohol jedoch wird hierbei ein Ni-Katalysator in Gegenwart von K₂CO₃ eingesetzt, wobei das K₂CO₃ die eigentliche Aktivität und Selektivität des Katalysators bedingt. Ohne Anwesenheit des K₂CO₃s wird bevorzugt tert. Amin hergestellt, obwohl ein Überschuss an Alkohol vorhanden ist.

Es bestand die Aufgabe, ein selektives Verfahren zur Herstellung von sekundären Aminen in der Flüssigphase bereit zu stellen, bei dem zum einen keine toxischen Katalysatoren eingesetzt werden müssen, eine lange Standzeit des Katalysators gewährleistet ist und zum anderen das sekundäre Amin sowohl in hohen Ausbeuten als auch mit hohen Selektivitäten erhalten wird. Eine weitere Aufgabe bestand in der Bereitstellung eines Verfahrens bei dem eine einfache und ergiebige Aufarbeitung des hergestellten sekundären Amins aus dem Reaktionsaustrag möglich ist.

Diese Aufgabe wird gelöst durch ein Verfahren zur Herstellung von sekundären Aminen der Formel I 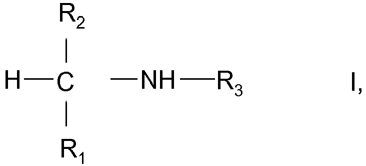 bei denen
R₁ und R₂ unabhängig von einander ausgewählt sind aus der Gruppe von Wasserstoff, linearen oder verzweigten aliphatischen Resten mit 1 bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit 1 bis 4 Kohlenstoffatomen substituiert sein können und R₃ ausgewählt ist aus der Gruppe von linearen oder verzweigten aliphatischen Resten mit 1 bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit 1 bis 4 Kohlenstoffatomen substituiert sein können

I) durch Aminierung von Alkoholen der Formel II 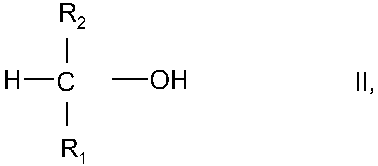 in denen R₁ und R₂ die oben genannte Bedeutung besitzen,
II) mit primären Aminen der Formel III

   R₃-NH₂ III,

   in der R₃ die oben genannte Bedeutung besitzt,
III) und Wasserstoff in der Flüssigphase in Gegenwart von Hydrierkatalysatoren, umfassend die folgenden Schritte:
   (i) Zuführung von Alkoholen der Formel II, primären Aminen der Formel III, Wasserstoff und gegebenenfalls Lösungsmittel in den Hydrierreaktor, wobei das Molverhältnis von Alkohol II zu primärem Amin III 1 bis 20 zu 1 beträgt,
   (ii) Durchführung der Hydrierung bei Temperaturen von 150 bis 210 °C und Drucken von 1 bis 300 bar in Gegenwart eines Hydrierkatalysators der Kupfer auf oxidischen Trägern enthält.

Bevorzugt ist das erfindungsgemäße Verfahren, wenn sekundäre Amine der Formel I, die aus 1-Dodecanol als Verbindung der Formel II mit Monomethylamin als Verbindung der Formel III hergestellt werden, ausgenommen sind.

Bevorzugt ist das erfindungsgemäße Verfahren, wenn R₁, R₂ und R₃ ausgewählt sind aus der Gruppe von linearen oder verzweigten aliphatischen Resten mit 1 bis 4 Kohlenstoffatomen.

Bevorzugt ist das erfindungsgemäße Verfahren, wenn der Kupferoxide enthaltende Katalysatorvorläufer 1 bis 80 Gew.-% Kupferoxid enthält.

Bevorzugt ist das erfindungsgemäße Verfahren, wenn als Katalysator-Träger Aluminiumoxide, Siliziumdioxid, Titandioxide, Zirkondioxid, Lanthanoxid, Molybdänoxid, Wolframoxid oder Gemische dieser Oxide verwendet werden.

Bevorzugt ist das erfindungsgemäße Verfahren, wenn Hydrierkatalysatoren, die neben Kupfer noch Wolfram und/oder Molybdän als Metall und/oder in Form ihrer Oxide enthalten, ausgenommen sind.

Bevorzugt ist das erfindungsgemäße Verfahren, wenn man als Katalysator-Träger Aluminiumoxid und/oder Lanthanoxid verwendet.

Bevorzugt ist das erfindungsgemäße Verfahren, wenn das Molverhältnis von Alkohol II zu primärem Amin III 1,5 bis 15 zu 1 beträgt.

Erfindungsgemäße Verfahren, wobei der Hydrieraustrag nach Schritt (ii) folgenden Aufarbeitungsschritten unterzogen wird:
(a) Destillative Abtrennung von Alkohol II, gegebenenfalls Amin der Formel III und einem Teil des Wassers aus dem Hydrieraustrag
(b) Extraktion des aus (a) erhaltenen, von Alkohol II, gegebenenfalls Amin der Formel III und einem Teil des Wassers befreiten Hydrieraustrags mit einer wässrigen Alkali- und/oder Erdalkalihydroxidlösung
(c) Abtrennung der aus Schritt (b) erhaltenen wässrigen Phase von der organischen Phase
(d) fraktionierte Destillation der aus Schritt c) erhaltenen organischen Phase unter Erhalt von sekundärem Amin I und
(e) Rückführung von gegebenenfalls Wasser enthaltendem Alkohol II und/oder Amin der Formel III in Hydrierschritt (ii)

Bevorzugt ist das erfindungsgemäße Verfahren, wenn das sekundäre Amin der Formel I N-Ethyl-tert.-butylamin ist.

Die erfindungsgemäße Umsetzung von Ethanol mit tert.-Butylamin und Wasserstoff zu Ethyl-tert.-Butylamin lässt sich durch die folgende Formelgleichung beschreiben:

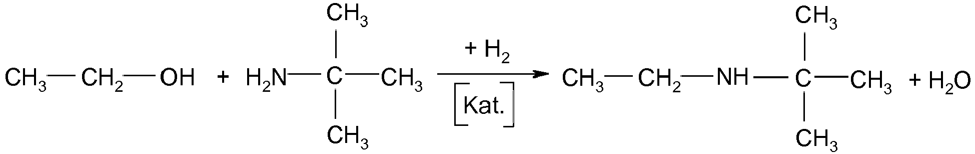

Die Aminierung wird diskontinuierlich oder kontinuierlich in der Flüssigphase durchgeführt. Bevorzugt ist ein kontinuierliches Verfahren.

Zur Herstellung der Amine der Formel I werden in einen Hydrierreaktor Wasserstoff, Alkohole der Formel II und Amine der Formel III gegebenenfalls mit einem Lösungsmittel gegeben.

Die Reste R₁ und R₂ in den Verbindungen der Formel I und II sind in dem erfindungsgemäßen Verfahren ausgewählt aus der Gruppe von Wasserstoff, linearen oder verzweigten aliphatischen Resten mit einem bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit einem bis 4 Kohlenstoffatomen substituiert sein können Bevorzugt sind die Aralkyl- oder Phenylreste mit aliphatischen Resten ausgewählt aus der Gruppe von Methyl-, Ethyl-, n-Propyl, i-Propyl-, n-Butyl-, i-Butyl, sek.-Butyl- und/oder tert.-Butylgruppen substituiert. R₃ in den Verbindungen der Formel I und III ist bevorzugt ausgewählt aus der Gruppe von linearen oder verzweigten aliphatischen Resten mit einem bis 15 Kohlenstoffatomen, cycloaliphatischen Resten mit 5 bis 10 Kohlenstoffatomen, Aralkylresten oder Phenylresten, die in o-, mund/oder p-Stellung durch aliphatische Reste mit einem bis 4 Kohlenstoffatomen substituiert sein können. Bevorzugt sind die Aralkyl- oder Phenylreste mit aliphatischen Resten ausgewählt aus der Gruppe von Methyl-, Ethyl-, n- Propyl, i-Propyl-, n-Butyl-, i-Butyl, sek.-Butyl- und/oder tert.-Butylgruppen substituiert.

Bevorzugte primäre Alkohole der Formel II sind ausgewählt aus der Gruppe von Methanol, Ethanol, n-Propanol, n-Butanol, 2-Methyl-1-propanol, Pivalinalkohol, n-Pentanol, n-Hexanol, 2-Ethylhexanol, 2-Methyl-1-pentanol, 3-Methyl-1-pentanol, 4-Methyl-1-pentanol, n-Octanol, n-Decanol, n-Undecanol, n-Dodecanol, 2-Phenylethanol, 2-Cyclopentylethanol, 2-Cyclohexylethanol, 2-Cycloheptylethanol, Methylphenylethanol, Benzylalkohol, Methylbenzylalkohol oder Gemischen dieser Alkohole.

Besonders bevorzugte primäre Alkohole der Formel II sind ausgewählt aus der Gruppe von Methanol, Ethanol, n-Propanol, n-Butanol, 2-Methyl-1-propanol oder Gemischen dieser Verbindungen.

Ganz besonders bevorzugt sind primäre Alkohole der Formel II ausgewählt aus der Gruppe Methanol, Ethanol, n-Propanol, n-Butanol oder Gemischen dieser Alkohole. Insbesondere ganz besonders bevorzugt ist Ethanol.

Bevorzugte sekundäre Alkohole der Formel II sind Isopropylalkohol, 2-Butanol, Cyclopentanol und Cyclohexanol.

Besonders bevorzugte sekundäre Alkohole der Formel II sind Isopropanol und Cyclohexanol.

Bevorzugte Amine der Formel III sind Methylamin, Ethylamin, n-Propylamin, i-Propylamin, n-Butylamin, 2-Methyl-1-propylamin, 2-Butylamin, tert.-Butylamin, n-Hexylamin, n-Decylamin, Anilin, Adamanthylamin, Dodecylamin, Benzylamin. Besonders bevorzugte Amine der Formel III sind tert.-Butylamin und Adamantylamin.

Besonders bevorzugt ist das sekundäre Amin der Formel I, das durch Umsetzung von 1-Do-decanol als Alkohol der Formel II mit Methylamin als Amin der Formel III nach dem erfindungsgemäßen Verfahren hergestellt wird ausgenommen.

Die Aminierung der Alkohole der Formel II wird erfindungsgemäß bei Temperaturen von 150 bis 210°C, bevorzugt 160 bis 200°C, besonders bevorzugt 170 bis 200°C durchgeführt.

Nach der Stöchiometrie der Aminierung ist ausgehend von Alkoholen kein Wasserstoff erforderlich. Vorteilhaft ist es jedoch, wenn Wasserstoff zugeführt wird.

Der Gesamtdruck im Reaktor bei der jeweiligen Temperatur setzt sich aus den Partialdrücken der Einsatzstoffe und der Reaktionsprodukte, also Wasserstoff, Alkohol der Formel II, Amin der Formel III, sekundärem Amin I, Wasser und gegebenenfalls einem mitverwendeten Lösungsmittel zusammen. Durch Aufpressen von Wasserstoff wird der Druck auf den gewünschten Reaktionsdruck erhöht. Um den Verbrauch an Wasserstoff auszugleichen, wird der Gesamtdruck während der Reaktionszeit durch Nachpressen von Wasserstoff konstant gehalten.

Der Gesamtdruck beträgt 1 bis 300 bar, bevorzugt 20 bis 250 bar, besonders bevorzugt 25 bis 250 bar, ganz besonders bevorzugt 30 bis 150 bar.

Das Molverhältnis von Alkohol der Formel II zu primärem Amin der Formel III beträgt 1 bis 20 zu 1, bevorzugt 1,5 bis 15 zu 1, besonders bevorzugt 2 bis 10 zu 1, ganz besonders bevorzugt 3 bis 5 zu 1. Durch die Wahl dieses Molverhältnisses wird eine hohe Selektivität an sekundärem Amin I erzielt.

Es kann vorteilhaft sein, das erfindungsgemäße Verfahren in Gegenwart eines unter den Reaktionsbedingungen inerten Lösungsmittels durchzuführen. Diese inerten Lösungsmittel sind dabei ausgewählt aus der Gruppe von N-Methylpyrrolidon oder Ethern wie Tetrahydrofuran, Dioxan, Ethylenglykoldimethylether oder Kohlenwasserstoffen wie Toluol, o-, m-, p-Xylol oder Gemischen dieser Lösungsmittel.

Bevorzugt ist jedoch, in Abwesenheit eines Lösungsmittels zu arbeiten.

Die Katalysator-Belastung liegt im Allgemeinen im Bereich von 0,01 bis 2, bevorzugt 0,05 bis 1, besonders bevorzugt 0,1 bis 0,5 kg primärem Amin der Formel III pro Liter Katalysator (Schüttvolumen) und Stunde.

Bevorzugt für das erfindungsgemäße Verfahren unter Verwendung von Alkoholen der Formel II und primären Aminen der Formel III sind Kupferoxide enthaltende Katalysator-Vorläufer, wobei das Kupferoxid auf oxidische Träger aufgebracht ist. Die Menge an Kupferoxid, gerechnet als CuO, beträgt dabei 1 bis 80 Gew.-%, bevorzugt 10 bis 70 Gew.-%, besonders bevorzugt 20 bis 65 Gew.-%, bezogen auf die Gesamtmasse des oxidischen Katalysator-Vorläufers. Dieser Katalysator-Vorläufer wird entweder vor der Hydrierung oder in der Anfangsphase der Hydrierung in Gegenwart von Alkoholen II und primären Aminen III zu elementarem Kupfer hydriert. Als Katalysator-Träger sind z. B. Aluminiumoxid, Siliziumdioxid, Titandioxid, Zirkondioxid, Lanthanoxid, Wolframoxid, Molybdänoxid und/oder Aktivkohle geeignet. Unter katalytisch aktiver Masse ist in diesem Zusammenhang die Summe aus Sauerstoff enthaltenden Kupferverbindungen und oxidischen Trägern zu verstehen.

Besonders bevorzugte, Kupferoxid auf Aluminiumoxid enthaltende Katalysator-Vorläufer lassen sich nach DE 3027890, Beispiele 1 a und 2 a herstellen. Die Formkörper besitzen eine spezifische Oberfläche von 50 bis 120 m² pro Gramm, enthalten ganz oder teilweise Kristalle mit Spinellstruktur und Kupfer in Gestalt von Kupferoxid. Man erhält diese Katalysator-Vorläufer dadurch, dass man Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 fällt und die so erhaltene Fällung bei einer Temperatur von 300 bis 800 °C calciniert.

Ebenfalls besonders bevorzugte, Kupferoxid auf oxidischen Materialien umfassend Aluminiumoxid und mindestens eines der Oxide des Lanthans, Wolframs, Molybdäns, Titans oder Zirkoniums, werden nach WO 2004/085356 A1 , Beispiel 1 hergestellt. Dem oxidischen Material werden pulverförmiges, metallisches Kupfer, Kupferblättchen, pulverförmiger Zement, Graphit oder ein Gemisch davon zugegeben. Gemische aus diesen Komponenten werden zu einem Formkörper verformt. Das oxidische Material enthält 50 bis 80 Gew.-%, vorzugsweise 55 bis 75 Gew.-% Kupferoxid, 15 bis 35 Gew.-%, vorzugsweise 20 bis 30 Gew.-% Aluminiumoxid und mindestens eines der Oxide des Lanthans, Wolframs, Molybdäns, Titans oder Zirkoniums in einer Menge von 2 bis 20, bevorzugt 3 bis 15 Gew.-%.

Die katalytisch aktive Masse der im erfindungsgemäßen Verfahren eingesetzten Katalysatoren kann weiterhin ein oder mehrere Elemente der Oxidationsstufe 0 oder deren anorganische oder organische Verbindungen, ausgewählt aus den Gruppen I A bis VI A und I B bis VII B und VIII des Periodensystems der Elemente enthalten.

Die Herstellung von geträgerten Kupfer-Katalysatoren ist in den Anmeldungen DE 3027890 A1 und WO 2004/085356 A1 detailliert beschrieben. Der Inhalt dieser Anmeldungen wird in vollem Umfang in die vorliegende Anmeldung einbezogen.

Als Reaktoren dienen bevorzugt Rohrreaktoren, wobei das erfindungsgemäße Verfahren in der Frischgasfahrweise oder der Kreisgasfahrweise durchgeführt werden kann. Bevorzugt wird es jedoch in Kreisgasfahrweise durchgeführt wird. Unter Kreisgasfahrweise ist dabei zu verstehen, dass unumgesetzter Wasserstoff nicht aus dem Verfahren ausgeschleust, sondern zusammen mit unter den Reaktionsbedingungen der Hydrieraustrags-Kondensation gasförmigen Verbindungen in den Hydrierreaktor zurückgeführt wird. Es ist aber auch möglich, überschüssigen Wasserstoff anderweitig zu nutzen oder zu verbrennen.

Die oxidischen Katalysator-Vorläufer werden gemahlen, mit Formhilfsmitteln vermischt, zu Tabletten, Kugeln, Ringen oder Strängen verformt und entweder außerhalb oder im Reaktor mit Wasserstoff reduziert und im Reaktor fest angeordnet.

Die Edukte werden in flüssiger Form kontinuierlich in Sumpf- oder Rieselfahrweise über den im Reaktor befindlichen Katalysator geleitet.

Es ist auch möglich, das erfindungsgemäße Verfahren in einem Wirbelbett mit in auf- und abwirbelnder Bewegung befindlichem Katalysatormaterial durchzuführen.

Vorteilhaft ist es, den Hydrieraustrag erfindungsgemäß aufzuarbeiten. Der nach Kühlung und Entspannung anfallende flüssige Hydrieraustrag enthält neben dem Zielprodukt, dem sekundären Amin der Formel I, als Nebenprodukte geringe Mengen an tertiärem Amin der Formel IV 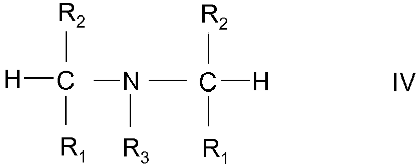 weiterhin überschüssigen Alkohol der Formel II und gegebenenfalls geringe Mengen an primärem Amin der Formel III. Unter geringen Mengen sind dabei jeweils weniger als 10 Gew.-%, bevorzugt weniger als 5 Gew.-%, besonders bevorzugt weniger als 1 Gew.-% der jeweils genannten Verbindungen zu verstehen. Bei der Aminierung entstehen etwa 5 bis 20 Gew.-% Wasser, bezogen auf die Menge des katalysatorfreien Hydrieraustrags. Sekundäre Amine der Formel I, primäre Amine der Formel III und Alkohole der Formel II können mit Wasser Azeotrope bilden. Daher lassen sich destillativ nur Gemische aus dem Hydrieraustrag abtrennen, die Reaktionswasser, Amin der Formel I, Amin der Formel II und/oder Alkohol der Formel II enthalten.

In EP-B 1312599 und EP-B 1312600 ist die Auftrennung von aminhaltigen Gemischen beschrieben, die ein oder mehrere Amine, Wasser, Leichtsieder und Schwersieder enthalten. Die Auftrennung erfolgt durch
(i) die destillative Abtrennung von Leichtsiedern von dem aminhaltigen Gemisch,
(ii) gegebenenfalls destillative Abtrennung von Schwersiedern von dem aminhaltigen Gemisch,
(iii) Extraktion des aminhaltigen Gemisches mit Natronlauge unter Gewinnung einer wässrigen, Natronlauge enthaltenden ersten Phase und einer wässrig-organischen, Amin enthaltenden zweiten Phase,
(iv) Destillation der wässrig-organischen zweiten Phase unter Gewinnung von Amin/Wasser-Azeotrop und von im wesentlichen wasserfreien Amin, und Rückführung des Amin/Wasser-Azeotrops in den Extraktionsschritt (iii).

Das im Wesentlichen wasserfreie Amin kann, falls erforderlich, destillativ weiter gereinigt werden. In einem Ausführungsbeispiel werden die Teilschritte der Aufarbeitung an einem Hydrieraustrag demonstriert, der durch reduktive Aminierung von 1,5-Pentandiol mit Ammoniak unter Bildung von Piperidin erhalten wurde.

Bevorzugt erfolgt für das erfindungsgemäße Verfahren ebenfalls eine Aufarbeitung des Hydrieraustrags, die sowohl Destillationen als auch die Brechung von Amin/Wasser- bzw. Alkohol/Wasser-Azeotropen mit wässrigen Alkali- und/oder Erdalkalihydroxidlösungen umfasst. Sowohl die Destillationen als auch die Brechung der Azeotrope können diskontinuierlich oder kontinuierlich durchgeführt werden.

Im Gegensatz zur Lehre von EP-B 1312599 und EP-B 1312600 erfolgt beim erfindungsgemäßen Verfahren zunächst die destillative Abtrennung von überschüssigem Alkohol, Reaktionswasser und gegebenenfalls nicht umgesetztem Amin der Formel III von dem restlichen Reaktionsaustrag. Je nach der Struktur des Alkohols können Alkohol und Wasser getrennt oder als trennbares Gemisch, als Homoazeotrop oder als Heteroazeotrop abgetrennt werden.

Da Methanol und Wasser kein Azeotrop bilden, kann Methanol getrennt oder zusammen mit dem Wasser abgetrennt werden. Erfolgt die Abtrennung gemeinsam, so können Methanol und Wasser anschließend in einer weiteren Kolonne getrennt werden. Das Methanol kann in die Aminierung zurückgeführt werden.

Da z. B. Ethanol ein Homoazeotrop mit Wasser bildet, destilliert ein Gemisch aus Ethanol und Wasser ab (bei Normaldruck 95,6 Gew.-% Ethanol und 4,4 Gew.-% Wasser). Da der Wassergehalt des Azeotrops gering ist, kann dieses ohne Abtrennung des Restwassers in die erfindungsgemäße Aminierung zurückgeführt werden.

Längerkettige Alkohole der Formel II, wie z. B. Butanole, bilden mit Wasser Heteroazeotrope, besitzen also eine Mischungslücke. Durch Phasentrennung kann der Alkohol vom Wasser abgetrennt und in die Aminierung zurückgeführt werden.

Bei der erfindungsgemäßen Aminierung entsteht ein Mol Wasser gemäß Reaktionsgleichung. Daher können Destillationsfraktionen, die Wasser, Alkohole der Formel II und/oder primäre Amine der Formel III enthalten, in die Synthesestufe (ii) des erfindungsgemäßen Verfahrens zurückgeführt werden,

Sofern nicht das gesamte Reaktionswasser zusammen mit den Alkoholen II aus dem Hydrieraustrag abgetrennt wurde, kommt es bei der weiteren destillativen Aufarbeitung zur Azeotropbildung zwischen sekundärem Amin und Wasser.

Aus den von Alkohol II und nur einem Teil des Reaktionswassers befreiten Hydrierausträgen muss das Restwasser entfernt werden. Dies erfolgt durch Behandlung des Hydrieraustrags mit wässriger, Alkali- und/oder Erdalkalihydroxidlösung. Die Alkali- und/oder Erdalkalihydroxidkonzentration in der wässrigen Lösung kann 1 bis 75 Gew.-%, bevorzugt 25 bis 50 Gew.-%, betragen. Bevorzugte wässrige Alkali- und/oder Erdalkalihydroxidlösungen sind ausgewählt aus der Gruppe von Natronlauge, Kalilauge, Magnesiumhydroxid, Calciumhydroxid. Bevorzugt ist Natronlauge. Besonders bevorzugt ist eine 50 Gew.-%ige Natronlauge.

Nach Extraktion des Hydrieraustrags mit der wässrigen Alkali- und/oder Erdalkalihydroxidlösung wird diese durch Phasentrennung abgetrennt. Der Restwassergehalt der organischen Phase kann zum Beispiel durch Karl-Fischer-Titration ermittelt werden. Die für die Wasserabtrennung benötigte Menge an Alkali- und/oder Erdalkalihydroxidlösung lässt sich durch wenige Vorversuche bestimmen.

Die für die Extraktion mit Alkali- und/oder Erdalkalihydroxidlösung eingesetzte Extraktionsvorrichtung kann ein- oder mehrstufig ausgebildet sein, beispielsweise ein einzelner Mixer-Settler-Extraktor. Mehrstufige Extraktionen sind beispielsweise Extraktionskolonnen oder Extraktionskaskaden. Als Extraktionskolonnen eignen sich beispielsweise Füllkörper-, Siebboden-, Kaskaden-, Pulsations-, Rotations- und Zentrifugalkolonnen. Eine Extraktionskaskade sind beispielsweise mehrere hintereinander geschaltete Mixer-Settler-Extraktoren, die auch platzsparend als Turmextraktor oder Kastenextraktor ausgeführt sein können. Ist der Extraktor mehrstufig, so ist bevorzugt eine Gegenstrom-Extraktionskolonne mit im allgemeinen 1 bis 25, bevorzugt 4 bis 10 theoretischen Trennstufen bevorzugt. Diese wird im allgemeinen bei einem Druck betrieben, bei dem alle Komponenten des Extraktionsgemisches unterhalb ihres Siedepunktes vorliegen, und sich ferner eine Viskosität der beiden Phasen einstellt, bei der eine Dispergierung der beiden Phasen problemlos möglich ist. Die Temperatur beträgt im Allgemeinen 5 bis 200°C, bevorzugt 20 bis 70°C, besonders bevorzugt 40 bis 50°C. Nach Phasentrennung wird die wässrige Alkali- und/oder Erdalkalihydroxidlösung enthaltende Phase aus dem Verfahren ausgeschleust.

Falls die abgetrennte wässrige Alkali- und/oder Erdalkalihydroxidlösung wesentliche Mengen an sekundärem Amin der Formel I, Alkohol der Formel II und/oder primärem Amin der Formel III enthält, so können diese Verbindungen durch Extraktion mit organischen Lösungsmitteln zurück gewonnen werden. Wesentliche Mengen liegen dann vor, wenn die Summe der obigen Verbindungen mehr als 10 Gew.-%, bevorzugt mehr als 5 Gew.-%, besonders bevorzugt mehr als 2 Gew.-% , bezogen auf den wasser- und katalysatorfreien Hydrieraustrag beträgt.

Als organische Lösungsmittel kommen dabei zum Beispiel aliphatische, cycloaliphatische oder aromatische Kohlenwasserstoffe in Frage, die mit wässriger Alkali- und/oder Erdalkalihydroxidlösung eine Mischungslücke besitzen. Beispiele für derartige Kohlenwasserstoffe sind n-Hexan, n-Octan, Cyclohexan, Toluol und Ethylbenzol oder Gemische dieser Verbindungen.

Die wässrige Alkali- und/oder Erdalkalihydroxidlösungsphase wird von der Kohlenwasserstoffphase durch Phasentrennung abgetrennt. Aus der Kohlenwasserstoffphase wird der Kohlenwasserstoff destillativ entfernt. Das zurück gewonnene sekundäre Amin der Formel I, der Alkohol der Formel II und/oder das primäre Amin der Formel III kann mit der Hauptmenge an rohem sekundärem Amin der Formel I, das aus der ersten organischen Phase nach Phasenabtrennung durch Extraktion gewonnen wurde vereinigt und destillativ aufgereinigt werden.

Es ist weiterhin möglich, das gegebenenfalls entstehende Azeotrop aus sekundärem Amin der Formel I und Wasser durch Zugabe von Kohlenwasserstoffen zum Hydrieraustrag, Abdestillieren von Kohlenwassertoff/Wasser-Heteroazeotropen aus dem Hydrieraustrag, Abtrennen der Wasser- von der Kohlenwasserstoffphase und Rückführung der Kohlenwasserstoffphase in die Destillation zu brechen.

Der wasserfreie oder nur noch weniger als 5, bevorzugt weniger als 3, besonders bevorzugt weniger als 1 Gew.-% Wasser enthaltende Hydrieraustrag kann durch fraktionierende Destillation weiter aufgereinigt werden. Die Destillation kann, abhängig von den zu destillierenden Mengen, kontinuierlich oder diskontinuierlich durchgeführt werden. Dabei geht zunächst, falls vorhanden, nicht umgesetztes primäres Amin III über Kopf ab. Dann folgt das Amin der Formel I, das ebenfalls über Kopf destilliert wird. Im Sumpf verbleiben, falls vorhanden, tertiäre Amine der Formel IV und Hochsieder. Nach GC-Analyse weniger als 97 Flächen-%, besonders bevorzugt weniger als 98 Flächen-%, besonders bevorzugt weniger als 99 Flächen-% sekundäres Amin I enthaltende Fraktionen können, falls eine Reinheit von über 97 Flächen-% erwünscht ist, in die Destillation zurückgeführt werden.

Für die fraktionierende Destillation kommen übliche Apparaturen in Betracht, wie sie beispielsweise in Kirk-Othmer, Encyclopedia of Chemical Technology, 3. Edition, Volume 7, John Wiley and Sons, New York, 1979, Seiten 870 bis 881 beschrieben sind. Bevorzugt sind dabei Siebbodenkolonnen, Glockenbodenkolonnen, Packungskolonnen oder Füllkörperkolonnen.

Durch fraktionierende Destillation werden Reinheiten der sekundären Amine der Formel I von mehr als 98 Flächen-%, insbesondere mehr als 99 Flächen-%, besonders bevorzugt von mehr als 99,5 Flächen-%, insbesondere mehr als 99,9, erzielt (GC-Analyse).

Für die kontinuierliche Aufarbeitung nach der Offenbarung EP-B 1312599 und EP-B 1312600, werden drei bis vier maßgeschneiderte Destillationskolonnen und eine Extraktionsvorrichtung benötigt.

Bei der erfindungsgemäßen diskontinuierlichen Aufarbeitung werden dagegen nur ein bis zwei Destillationskolonnen und eine Extraktionsvorrichtung verwendet.

### Beispiele

Allgemeine Arbeitsvorschrift für die Aminierung von primären und sekundären Alkoholen mit primären Aminen in der Flüssigphase.

### Katalysator-Herstellung

Die Herstellung des Kupfer-Katalysator-Vorläufers erfolgte nach DE 3027890, Beispiel 1 a. Er besaß die Zusammensetzung 55 Gew.-% CuO und 45 Gew.-% gamma-Al₂O₃ in Form von 3 x 3 mm Tabletten. Der Kupfer-Katalysator-Vorläufer wurde nach Beispiel 1 b mit Wasserstoff reduziert und unterhalb von 50 °C mit Luft passiviert (Katalysator A). Ein weiterer Kupfer-Katalysator-Vorläufer erfolgt nach WO-A1 04/085356, Beispiel 1. (Katalysator B)

### Versuchsdurchführung (Batchfahrweise)

Die Aminierung wurde in einem magnetgekuppelten 300 ml Rührautoklaven mit Elektroheizung und Innentemperatur-Kaskadenregelung durchgeführt. In den mit Stickstoff inertisierten Autoklaven wurde die jeweilige Menge an primärem oder sekundärem Alkohol, primärem Amin und Kupfer-Katalysator in reduzierter und passivierter Form eingefüllt. Auf das Reaktionsgemisch wurde bei Raumtemperatur Wasserstoff bis zu einem Druck von 10 bar aufgepresst. Dann wurde das Reaktionsgemisch auf die vorgesehene Reaktionstemperatur erhitzt, Wasserstoff bis zu dem vorgesehenen Gesamtdruck nachgepresst und die vorgesehene Reaktionszeit unter diesen Bedingungen gerührt. Die Rührerdrehzahl betrug 700 rpm.

Nach der vorgesehenen Reaktionszeit wurde das Reaktionsgemisch abgekühlt und entspannt. Die Ausbeute an den Zielprodukten, den sekundären Aminen I, den nicht umgesetzten primären Aminen III und den tertiären Aminen als Nebenprodukten wurde gaschromatographisch ermittelt (30 m RTX 5 Amin-Säule). Bei den Ausbeute-Angaben handelt es sich um GC-Flächenprozente. Die sek.-Aminselektivität (S) wird definiert als Flächen% _{sek. Amin}: (100 - Flächen% _{prim.Amin})*100.

### Beispiele 1 bis 5

### Aminierung von Ethanol mit tert.-Butylamin zu Ethyl-tert.-butylamin (Batchfahrweise)

Tabelle 1 enthält die Mengen an Ausgangsverbindungen und die Reaktionsbedingungen. In allen Versuchen wurden 10 g des Kupfer-Katalysators A eingesetzt.

Nach zwei, sechs und 10 Stunden Reaktionszeit wurden Proben entnommen und gaschromatographisch auf tert.-Butylamin, Ethyl-tert.-butylamin und Diethyl-tert.-butylamin untersucht.

Tabelle 1 zeigt, dass zwischen 160 und 180 °C bei tert.-Butylamin/Ethanol-Molverhältnissen zwischen 1 zu 5 bis 1 zu 20 Ausbeuten an Ethyl-tert.-butylamin zwischen rund 88 und 91 % und Selektivitäten bis zu 97 % erzielt werden.

Besonders hervorzuheben ist Versuch 3, bei dem nach sechs Stunden Reaktionszeit 88,4 Flächen-% Ethyl-tert.-butylamin und nur 1,6 Flächen-% Diethyl-tert.-butylamin erhalten wurden. Das nicht umgesetzte tert.-Butylamin (8,7 Flächen%) lässt sich destillativ abtrennen und in die Synthese zurückführen. Die Selektivität beträgt 96,8% (Tabelle1).

### Beispiel 6

### Aminierung von i-Propanol mit Adamantylamin zu i-Propyl-adamantylamin (Batchfahrweise)

Ein Gemisch aus 6 g Adamantylamin, 96 g i-Propanol, 15.4 g o-Xylol und 8 g gemahlenem reduziertem und passiviertem Katalysator A wurde in einem inertisierten magnetgekuppelten 300 ml Autoklaven gerührt. Dann wurden 5 bar Wasserstoff aufgepresst. Das Gemisch wurde auf 200 °C erwärmt. Der Druck stieg auf 29 bar. Wasserstoff wurde bis zu einem Gesamtdruck von 40 bar nachgepresst. Dann wurde 6 Stunden lang bei 200°C und 40 bar Gesamtdruck gerührt. Alle zwei Stunden wurden Proben gezogen und gaschromatographisch analysiert.

Tabelle 2 zeigt, dass i-Propyl-adamantyl[amin]-Ausbeuten zwischen 90 und 92 % erzielt wurden.

### Beispiel 7

### Kontinuierliche Aminierung von Ethanol mit tert.-Butylamin zu Ethyl-tert.-butylamin

Die Hydrierung wurde 241 Stunden lang in einem Rohrreaktor (Länge 2500 mm, Durchmesser 30 mm) durchgeführt, in den 700 ml des Katalysator-Vorläufers (55 Gew.-% CuO, 45 Gew.-% Al₂O₃, 3 x 3 mm Tabletten) unter Stickstoff eingefüllt wurden. Die Herstellung des Katalysator-Vorläufers erfolgte nach DE 3027890 A1, Beispiel 1 a.

Die Reduktion des Katalysator-Vorläufers erfolgte drucklos, erst 6 Stunden bei 180°C, dann 6 Stunden bei 200°C mit Gemischen aus 20 NL Wasserstoff und 400 NL Stickstoff, dann 6 Stunden bei 200°C mit Gemischen aus 40 NL Wasserstoff und 400 NL Stickstoff und schließlich nach Umstellung innerhalb von 6 Stunden auf reinen Wasserstoff 6 Stunden lang mit 200 NL Wasserstoff.

Die Hydrierung wurde in Sumpffahrweise bei Temperaturen von 170 bis 185°C, Drücken von 50 bar und Molverhältnissen von tert.-Butylamin zu Ethanol von 1 zu 3 bis 1 zu 5 betrieben. Die Katalysator-Belastung betrug 0,15 bis 0,30 kg pro Liter Katalysator und Stunde.

Nach 228 Stunden wurde laut GC-Analyse folgendes Ergebnis erzielt (Ausbeute = GC-Flächen-%, Ethanol wurde aus der GC-Analyse herausgerechnet):

| | Flächen-% | |
|---|---|---|
| | nach 109 h | nach 228 h |
| • Ethyl-tert.-butylamin | 84,2 | 85,3 |
| • Diethyl-tert.-butylamin | 2,5 | 2,1 |
| • tert.-Butylamin | 13,0 | 12,5 |

Da tert.-Butylamin bei der Aufarbeitung zurück gewonnen werden kann, beträgt die Ethyl-tert.-butylamin-Selektivität nach 109 Stunden 97,5 %, nach 228 Stunden 96,8 %.

Die Reaktionsbedingungen nach 109 bzw. 228 Stunden Hydrierzeit waren 170°C und 50 bar. Das Molverhältnis von tert.-Butylamin zu Ethanol betrug 1 zu 3 (nach 109-121 Stunden 1 zu 10), die Katalysator-Belastung 0,25 g pro Liter Katalysator und Stunde.

Der Versuch zeigt, dass im Gegensatz zu US 4.206.150, Vergleichsbeispiel 6, keine Desaktivierung des Kupfer-Katalysators beobachtet wird.

### Beispiel 8 und 9

### Aminierung von Ethanol bzw. i-Propanol mit Cyclohexylamin

Die Aminierung wurde mit Ethanol bzw. i-Propanol und Cyclohexylamin in Gegenwart von 5 g gemahlenem reduziertem und passiviertem Katalysator A gemäss der allgemeinen Arbeitsvorschrift durchgeführt. Die Einsatzstoffmengen und Reaktionsbedingungen sind in Tabelle 3 enthalten.

Die GC-Analyse zeigt, dass eine hohe Selektivität der gewünschten sekundären Amine erzielt wurde. So beträgt die GC-Fläche des Ethyl-cyclohexylamins bzw. i-Propyl-cyclohexylamins zur Summe der Flächen aus sekundärem + tertiärem Amin 94% bzw. 99%.

Nicht umgesetztes Cyclohexylamin und nicht umgesetzte Alkohole können aus dem Hydrieraustrag abgetrennt und in die Synthesestufe zurückgeführt werden.

**Tabelle 1**

| Herstellung von Ethyl-tert.-butylamin (ETBA) | | | | | | | | | | | | | | | | | |
|---|---|---|---|---|---|---|---|---|---|---|---|---|---|---|---|---|---|
| 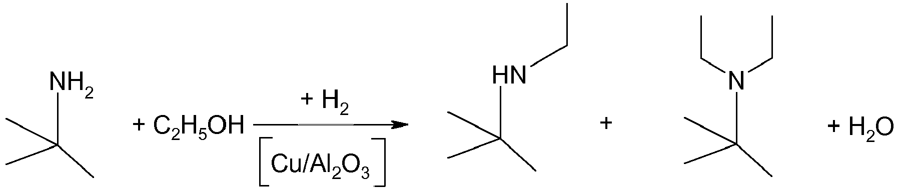 | | | | | | | | | | | | | | | | | |
| Versuch Nr. | Ethanol [g] | tert.-Butylamin [g] | Molverh. Amin zu Alkohol | Temperatur [°C] | Gesamtdruck [bar] | GC-Flächen-% 2 Stunden | | | | GC-Flächen-% 6 Stunden | | | | GC-Flächen-% 10 Stunden | | | |
| | | | | | | prim. ¹⁾ | sek.²⁾ | tert.³⁾ | S⁴⁾ | prim. | sek. | tert. | S⁴⁾ | prim. | sek. | tert. | S⁴⁾ |
| 1 | 92 | 7,3 | 1 : 20 | 160 | 80 | 36,3 | 57,9 | 0,2 | 90,9 | 0,7 | 89,6 | 6,3 | 90,3 | 0,1 | 78,5 | 17,6 | 78,1 |
| 2 | | 14,6 | 1 : 10 | | | 60,8 | 37,2 | 0 | 94,9 | 15,4 | 82,1 | 1 | 97,0 | 0,4 | 91,3 | 6,7 | 91,9 |
| 3 | | 29,2 | 1 : 5 | 170 | | 57,4 | 40,3 | 0,03 | 94,6 | 8,7 | 88,4 | 1,6 | 96,8 | 0,7 | 89,1 | 8,9 | 89,7 |
| 4 | | 7,3 | 1 : 20 | 180 | | 0,5 | 87,7 | 8,6 | 88,1 | 0,2 | 60,1 | 34,6 | 60,2 | 0,1 | 40,3 | 52,8 | 40,3 |
| 5 | | | | 210 | | 0 | 31,6 | 53,9 | 31,6 | 0 | 25,9 | 53,2 | 25,9 | 0 | 23,0 | 50,1 | 23,0 |

| | | | | | | | | | | | | | | | | | |
|---|---|---|---|---|---|---|---|---|---|---|---|---|---|---|---|---|---|
| 1) Tert.-Butylamin 2) Ethyl-tert.-butylamin 3) Diethyl-tert.-butylamin 4) ETBA-Selektivität [%] | | | | | | | | | | | | | | | | | |

**Tabelle 2**

| Herstellung von i-Propyladamantylamin | | | | |
|---|---|---|---|---|
| 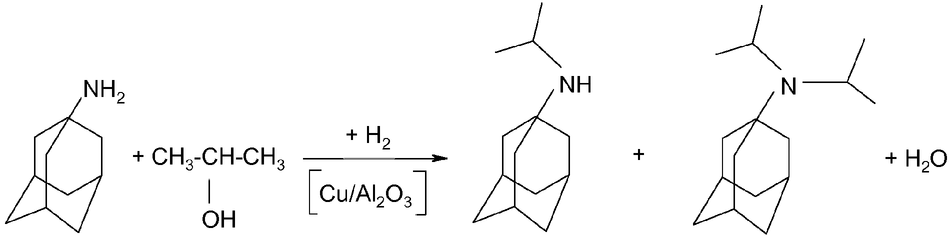 | | | | |
| Versuch Nr. | Reaktionszeit [h] | GC-Flächen-% | | |
| | | Adamantylamin | i-Propyladamantylamin | Di-i-Propyladamantylamin |
| 6 | 2 | 9,8 | 90,2 | - |
| | 4 | 6,4 | 91,3 | - |
| | 6 | 6,7 | 90,7 | - |

**Tabelle 3**

| 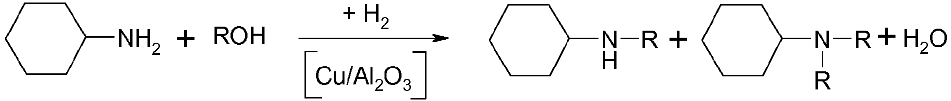 | | | | | | | | | | | | |
|---|---|---|---|---|---|---|---|---|---|---|---|---|
| Versuch Nr. | R | ROH [g] | Cyclohexylamin [g] | Molverh. Amin:ROH | T [°C] | Gesamtdruck | Reaktionszeit | GC-Flächen% | | | | |
| | | | | | | | | prim¹⁾ | sek²⁾ | tert³⁾ | ROH | N⁴⁾ |
| 8 | C₂H₅ | 100 | 43 | 1:5 | 170 | 80 | 6 | 13,1 | 34,8 | 2,1 | 45,2 | 4,8 |
| 9 | i-C₃H₇ | 105 | 35 | | | | | 0,9 | 42,0 | 0,2 | 50,0 | 6,9 |

| | | | | | | | | | | | | |
|---|---|---|---|---|---|---|---|---|---|---|---|---|
| 1) Cyclohexylamin 2) Alkylcyclohexylamin 3) Dialkylcyclohexylamin 4) N = Summe der Nebenkomponenten | | | | | | | | | | | | |

## Patentansprüche

1. Verfahren zur Herstellung von sekundären Aminen der Formel I 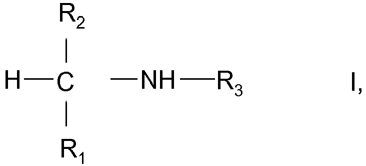 bei denen
R₁ und R₂ unabhängig voneinander ausgewählt sind aus der Gruppe von Wasserstoff, linearen oder verzweigten aliphatischen Resten mit 1 bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit 1 bis 4 Kohlenstoffatomen substituiert sein können und
R₃ ausgewählt ist aus der Gruppe von linearen oder verzweigten aliphatischen Resten mit 1 bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit 1 bis 4 Kohlenstoffatomen substituiert sein können,
I) durch Aminierung von Alkoholen der Formel II 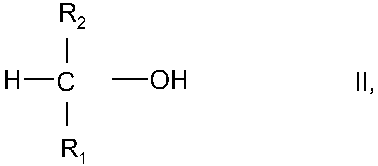 in denen R₁ und R₂ die oben genannte Bedeutung besitzen,
II) mit primären Aminen der Formel III
R₃-NH₂ III,
in der R₃ die oben genannte Bedeutung besitzt,
III) und Wasserstoff in der Flüssigphase in Gegenwart von Hydrierkatalysatoren, umfassend die folgenden Schritte:
(i) Zuführung von Alkoholen der Formel II, primären Aminen der Formel III, Wasserstoff und gegebenenfalls Lösungsmittel in den Hydrierreaktor, wobei das Molverhältnis von Alkohol II zu primärem Amin III 1 bis 20 zu 1 beträgt,
(ii) Durchführung der Hydrierung bei Temperaturen von 150 bis 210 °C und Drucken von 1 bis 300 bar in Gegenwart eines Hydrierkatalysators der Kupfer auf oxidischen Trägern enthält.

2. Verfahren nach Anspruch 1, wobei sekundäre Amine der Formel I die aus 1-Dodecanol als Verbindung der Formel II mit Monomethylamin als Verbindung der Formel II hergestellt werden, ausgenommen sind.

3. Verfahren nach einem der Ansprüche 1 bis 2, wobei R₁, R₂ und R₃ ausgewählt sind aus der Gruppe von linearen oder verzweigten aliphatischen Resten mit 1 bis 4 Kohlenstoffatomen.

4. Verfahren nach einem der Ansprüchen 1 bis 3, wobei der Kupferoxide enthaltende Katalysatorvorläufer 1 bis 80 Gew.-% Kupferoxid enthält.

5. Verfahren nach einem der Ansprüche 1 bis 4, wobei als Katalysator-Träger Aluminiumoxide, Siliziumdioxid, Titandioxide, Zirkondioxid, Lanthanoxid, Molybdänoxid, Wolframoxid oder Gemische dieser Oxide verwendet werden.

6. Verfahren nach einem der Ansprüche 1 bis 5, wobei Hydrierkatalysatoren, die neben Kupfer noch Wolfram und/oder Molybdän als Metall und/oder in Form ihrer Oxide enthalten, ausgenommen sind.

7. Verfahren nach einem der Ansprüche 1 bis 6, wobei man als Katalysator-Träger Aluminiumoxid und/oder Lanthanoxid verwendet.

8. Verfahren nach den Ansprüchen 1 bis 7, wobei das Molverhältnis von Alkohol II zu primärem Amin III 1,5 bis 15 zu 1 beträgt.

9. Verfahren nach Anspruch 1, wobei der Hydrieraustrag nach Schritt (ii) folgenden Aufarbeitungsschritten unterzogen wird:
(a) Destillative Abtrennung von Alkohol 11, gegebenenfalls Amin der Formel III und einem Teil des Wassers aus dem Hydrieraustrag
(b) Extraktion des aus (a) erhaltenen, von Alkohol 11, gegebenenfalls Amin der Formel III und einem Teil des Wassers befreiten Hydrieraustrags mit einer wässrigen Alkali- und/oder Erdalkalihydroxidlösung
(c) Abtrennung der aus Schritt (b) erhaltenen wässrigen Phase von der organischen Phase
(d) fraktionierte Destillation der aus Schritt c) erhaltenen organischen Phase unter Erhalt von sekundärem Amin I und
(e) Rückführung von gegebenenfalls Wasser enthaltendem Alkohol II und/oder Amin der Formel III in Hydrierschritt (ii)

10. Verfahren nach einem der Ansprüche 1 bis 8, wobei das sekundären Amin der Formel I N-Ethyl-tert-butylamin ist.

## Claims

1. A process for preparing secondary amines of the formula I 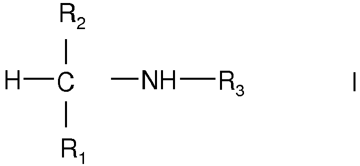 in which
R₁ and R₂ are each independently selected from the group of hydrogen, linear or branched aliphatic radicals having 1 to 15 carbon atoms, cycloaliphatic radicals having 5 to 10 carbon atoms, aralkyl radicals and phenyl radicals which may be o-, m- and/or p-substituted by aliphatic radicals having 1 to 4 carbon atoms, and
R₃ is selected from the group of linear or branched aliphatic radicals having 1 to 15 carbon atoms, cycloaliphatic radicals having 5 to 10 carbon atoms, aralkyl radicals and phenyl radicals which may be o-, m- and/or p-substituted by aliphatic radicals having 1 to 4 carbon atoms,
I) by aminating alcohols of the formula II 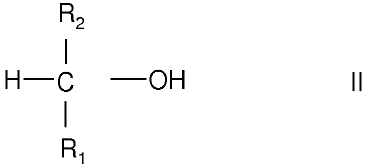 in which R₁ and R₂ are each as defined above
II) with primary amines of the formula III
R₃-NH₂ III
in which R₃ is as defined above
III) and hydrogen in the liquid phase in the presence of hydrogenation catalysts, comprising the following steps:
(i) feeding alcohols of the formula II, primary amines of the formula III, hydrogen and optionally solvent into the hydrogenation reactor, the molar ratio of alcohol II to primary amine III being 1 to 20:1,
(ii) performing the hydrogenation at temperatures of 150 to 210°C and pressures of 1 to 300 bar in the presence of a hydrogenation catalyst comprising copper on oxidic supports.

2. The process according to claim 1, excluding secondary amines of the formula I which are prepared from 1-dodecanol as the compound of the formula II with monomethylamine as the compound of the formula III.

3. The process according to either of claims 1 and 2, wherein R₁, R₂ and R₃ are each selected from the group of linear or branched aliphatic radicals having 1 to 4 carbon atoms.

4. The process according to any of claims 1 to 3, wherein the catalyst precursor comprising copper oxides comprises 1 to 80% by weight of copper oxide.

5. The process according to any of claims 1 to 4, wherein the catalyst support used comprises aluminum oxides, silicon dioxide, titanium dioxides, zirconium dioxide, lanthanum oxide, molybdenum oxide, tungsten oxide or mixtures of these oxides.

6. The process according to any of claims 1 to 5, excluding hydrogenation catalysts which, as well as copper, also comprise tungsten and/or molybdenum as a metal and/or in the form of the oxides thereof.

7. The process according to any of claims 1 to 6, wherein the catalyst support used is aluminum oxide and/or lanthanum oxide.

8. The process according to claims 1 to 7, wherein the molar ratio of alcohol II to primary amine III is 1.5 to 15:1.

9. The process according to claim 1, wherein the hydrogenation output after step (ii) is subjected to the following workup steps:
(a) distillatively removing alcohol II, optionally amine of the formula III and a portion of the water from the hydrogenation output,
(b) extracting the hydrogenation output which has been obtained from (a) and freed of alcohol II, optionally amine of the formula III and a portion of the water with an aqueous alkali metal and/or alkaline earth metal hydroxide solution,
(c) removing the aqueous phase obtained from step (b) from the organic phase, 4
(d) fractionally distilling the organic phase obtained from step c) to obtain secondary amine I and
(e) recycling optionally water-comprising alcohol II and/or amine of the formula III into hydrogenation step (ii).

10. The process according to any of claims 1 to 8, wherein the secondary amine of the formula I is N-ethyl-tert-butylamine.

## Revendications

1. Procédé de fabrication d'amines secondaires de formule I 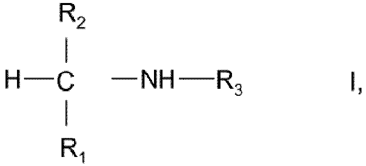 dans laquelle
R₁ et R₂ sont choisis indépendamment l'un de l'autre dans le groupe constitué par l'hydrogène, les radicaux aliphatiques linéaires ou ramifiés de 1 à 15 atomes de carbone, les radicaux cycloaliphatiques de 5 à 10 atomes de carbone, les radicaux aralkyle ou les radicaux phényle, qui peuvent être substitués en position o, m et/ou p par des radicaux aliphatiques de 1 à 4 atomes de carbone, et
R₃ est choisi dans le groupe constitué par les radicaux aliphatiques linéaires ou ramifiés de 1 à 15 atomes de carbone, les radicaux cycloaliphatiques de 5 à 10 atomes de carbone, les radicaux aralkyle ou les radicaux phényle, qui peuvent être substitués en position o, m et/ou p par des radicaux aliphatiques de 1 à 4 atomes de carbone,
I) par amination d'alcools de formule II 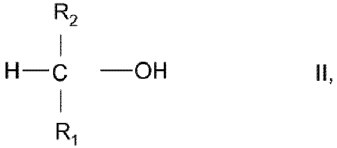 dans laquelle R₁ et R₂ ont la signification indiquée précédemment,
II) avec des amines primaires de formule III
R₃-NH₂ III,
dans laquelle R₃ a la signification indiquée précédemment,
III) et de l'hydrogène dans la phase liquide en présence de catalyseurs d'hydrogénation, comprenant les étapes suivantes :
(i) l'introduction d'alcools de formule II, d'amines primaires de formule III, d'hydrogène et éventuellement de solvants dans le réacteur d'hydrogénation, le rapport molaire entre l'alcool II et l'amine primaire III étant de 1 à 20 sur 1,
(ii) la réalisation de l'hydrogénation à des températures de 150 à 210 °C et des pressions de 1 à 300 bar en présence d'un catalyseur d'hydrogénation à base de cuivre sur des supports oxydiques.

2. Procédé selon la revendication 1, dans lequel les amines secondaires de formule I qui sont fabriquées à partir de 1-dodécanol en tant que composé de formule II avec de la monométhylamine en tant que composé de formule III sont exclues.

3. Procédé selon l'une quelconque des revendications 1 à 2, dans lequel R₁, R₂ et R₃ sont choisis dans le groupe constitué par les radicaux aliphatiques linéaires ou ramifiés de 1 à 4 atomes de carbone.

4. Procédé selon l'une quelconque des revendications 1 à 3, dans lequel le précurseur de catalyseur contenant des oxydes de cuivre contient 1 à 80 % en poids d'oxyde de cuivre.

5. Procédé selon l'une quelconque des revendications 1 à 4, dans lequel des oxydes d'aluminium, du dioxyde de silicium, des dioxydes de titane, du dioxyde de zirconium, de l'oxyde de lanthane, de l'oxyde de molybdène, de l'oxyde de tungstène ou des mélanges de ces oxydes sont utilisés en tant que support de catalyseur.

6. Procédé selon l'une quelconque des revendications 1 à 5, dans lequel les catalyseurs d'hydrogénation qui contiennent également du tungstène et/ou du molybdène en tant que métal et/ou sous la forme de leurs oxydes en plus du cuivre sont exclus.

7. Procédé selon l'une quelconque des revendications 1 à 6, dans lequel de l'oxyde d'aluminium et/ou de l'oxyde de lanthane sont utilisés en tant que support de catalyseur.

8. Procédé selon l'une quelconque des revendications 1 à 7, dans lequel le rapport molaire entre l'alcool II et l'amine primaire III est de 1,5 à 15 sur 1.

9. Procédé selon la revendication 1, dans lequel la sortie de l'hydrogénation selon l'étape (ii) est soumise aux étapes de traitement suivantes :
(a) la séparation par distillation de l'alcool II, éventuellement de l'amine de formule III et d'une partie de l'eau de la sortie de l'hydrogénation,
(b) l'extraction de la sortie de l'hydrogénation obtenue en (a), débarrassée de l'alcool II, éventuellement de l'amine de formule III et d'une partie de l'eau avec une solution aqueuse d'hydroxyde alcalin et/ou alcalino-terreux,
(c) la séparation de la phase aqueuse obtenue à l'étape (b) de la phase organique,
(d) la distillation fractionnée de la phase organique obtenue à l'étape c) pour obtenir l'amine secondaire I et
(e) le recyclage l'amine de formule III et/ou de l'alcool II contenant éventuellement de l'eau dans l'étape d'hydrogénation (ii).

10. Procédé selon l'une quelconque des revendications 1 à 8, dans lequel l'amine secondaire de formule I est la N-éthyl-tert-butylamine.
